# EUROPEAN PATENT APPLICATION

(11) **EP 2 878 323 A1**
(43) Date of publication of application: **03.06.2015**
(21) Application number: 13194897.8
(22) Date of filing: 28.11.2013
(51) Int. Cl.: A61M 5/32

(54) **Drug delivery device having a needle shield with needle cap remover**

(71) Applicant: Sanofi-Aventis Deutschland GmbH, 65929 Frankfurt am Main (DE)
(72) Inventor: The designation of the inventor has not yet been filed

(57) **Abstract**

The invention relates to a rug delivery device (1), comprising:
- a housing (2) containing and holding a container (6) with a needle (5) attachable at its distal end,
- a needle shield (4) adapted to be movable with respect to the housing (2) between an advanced position (PI) in which the needle (5) is covered and a retracted position (PII) in which the needle (5) extends through an distal opening (4.2) of the needle shield (4), and
- a protective needle cap (7) releasably attached to cover the needle (5) before injection, wherein the needle shield (4) is adapted to move in a double movement sequence (M1, M2) between the advanced position (PI) and the retracted position (PII) to perform different operations in sequence.

## Description

### Technical Field

The invention relates to a drug delivery device.

### Background of the Invention

Many drug delivery devices of the prior art, such as auto-injectors, syringes, have been developed for self-administration of the drug.

To protect the needle of the drug delivery device from damage or to protect people from needle-prick injuries before using of the device, the needle of the drug delivery device is covered by a protective needle cap or the so-called rigid needle shield (shortly named RNS).

In order to prepare the drug delivery device for delivering a dose the protective needle cap has to be removed from the needle. This may be done by gripping the protective needle cap and pulling it away from the needle. This will usually result in an exposed needle which is undesirable in terms of needle safety. In order to solve that problem the needle of the drug delivery device could be covered by a needle shield or shroud in a manner to hide the needle when the protective needle cap is removed.

### Summary of the Invention

It is an object of the present invention to provide a drug delivery device that reduces the risk of needle-prick injuries and needle damages in particular before using the device and which is simple to use, manufacture and assembling.

The object is achieved by a drug delivery device according to claim 1.

Preferred embodiments of the invention are given in the dependent claims.

According to the invention, a drug delivery device comprises a housing containing and holding a container with a needle attachable at its distal end, a needle shield adapted to be movable with respect to the housing between an advanced position in which the needle is covered and a retracted position in which the needle extends through a distal opening of the needle shield, and a protective needle cap releasably attached to cover the needle before injection, wherein the needle shield is adapted to move in a double movement sequence between the advanced position and the retracted position to perform different operations in sequence.

The invention allows removing the protective needle cap without any additional remover elements, e.g. manual by hand. Furthermore, the invention avoids an unintended activation of the needle shield due to the fact that a set-up activation of the needle shield is necessary to get it ready for injection.

In a possible embodiment of the invention, a first operation with a respective first movement sequence of the needle shield is provided. In the first operation and during the first movement sequence before injection, the needle shield is movable from the advanced or start position to the retracted position and back to the advanced or intermediate position, thereby the needle shield releases the protective needle cap. In particular during the first movement sequence, the needle shield is adapted to capture the protective needle cap at least in the retracted position and if the needle shield returns back to the advanced or starting position the protective needle cap will be taken along and thus away from the needle so that it can be manually removed or falls off. Thus, the invention allows a removing of the protective needle cap without any additional remover elements.

Furthermore, a second operation with a respective second movement sequence of the needle shield is provided. In the second operation and during the second movement sequence from the advanced or intermediate position to the retracted position and back to the advanced or end position, an injection is provided wherein the needle is covered by the needle shield before and after injection and extends through the opening of the needle shield only during injection. After the second movement sequence, the needle shield is securely and permanently fixed to the housing in the advanced or end position. Thus, the needle shield of the drug delivery device sequentially fulfils two operations by two similar succeeding push movement sequences of the needle shield, firstly push movement sequence for removing the protective needle shield and secondly push movement sequence for allowing injection including needle protection before and after injection.

According to another aspect of the invention, a guiding arrangement is disposed between the housing and the needle shield and guiding the needle shield with respect to the housing, wherein the guiding arrangement is adapted to guide the needle shield in the double movement sequence. The guiding arrangement allows an essential linear movement and thus a simple and safe guiding of the needle shield with respect to the housing for performing the different operations, namely the removing operation and the injection operation in sequence.

In one embodiment of the invention, the guiding arrangement has a labyrinth-shape with different strokes for the different movement sequences. Preferably, one stroke represents one operation and thus one push movement sequence of the needle shield so that the different movement sequences are separated from each other.

According to an additional feature of the invention, the guiding arrangement comprises a guide track and a guide arm, wherein the guide track is formed in the housing and the guide arm is articulated on the needle shield or vice versa. Preferably, the guide arm is flexible and comprises an end forming a pin which cooperates with the guide track. Further, the guide arm projects inwards the needle shield and is adapted to pivot about a vertical axis. Due to the flexible design of the guide arm, a rotation during the linear movement of the needle shield with respect to the housing is prevented. Thus, the comfort during injection is improved. The guide track comprises a plurality of recesses which are arranged essentially in parallel to one another and are separated from each other by ribs so as to form different stroke guides for the different movement sequences of the needle shield.

According to another feature of the invention, each of at least two of the recesses is designed as a labyrinth chamber with a closed end and an opposite opened end to form a respective stroke for one of the movement sequences. Due to the one-sided closed recesses, each recess forms a chamber in which the guide arm engages at the closed end in order to stop the movement of the needle shield with respect to the housing.

In detail, one of the ends of the recesses are closed and the opposite ends are opened in such a manner that the end of the guide arm engages one of the closed ends of the recesses in the different operations and, during one of the double movement sequences, it is guided from one of the recesses through the opened ends into an adjacent recess. Such a designed labyrinth-shape of the guiding arrangement allows a simple separation of the different operations and push movement sequences of the needle shield.

According to a still another embodiment, the recesses are slightly curved in the area of the closed end to form a releasable rest position.

According to another feature of the invention, in the area of a closed end of one of the recesses, at least one of the respective ribs of that recess comprises a hooked-shaped projection in which the end of the guide arm is securely and safely fixed to the housing in the advanced position. Thus, an easy and simple permanent locking of the needle shield with respect to the housing after injection in the advanced position in which the needle is covered is provided and a further movement of the needle shield is prevented.

According to a further feature of the invention, at least two guide arrangements are provided arranged opposite each other and synchronized to each other to support a safe guiding of the needle shield with respect to the housing.

Yet to a further embodiment, a snap arm is provided at the needle shield. The snap arm comprises at its free end a locking hook to capture the protective needle cap.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: shows in a side view an embodiment of a drug delivery device in a delivery state,
- Figure 2: shows in a side view the drug delivery device after a movement of the needle shield from an advanced position into a retracted position in which a protective needle cap extends through an opening of the needle shield,
- Figure 3: shows in a side view the drug delivery device with the needle shield back into the advanced position in which the protective needle cap further extends through the opening of the needle shield,
- Figure 4: shows in a side view the drug delivery device before an injection and with a needle shield in the advanced position and without protective needle cap,
- Figure 5: shows in a side view the drug delivery device during injection and with a needle shield in the retracted position so that the needle extends through the opening of the needle shield,
- Figure 6: shows a schematic view of an embodiment of a guiding arrangement with a guide track having a labyrinth-shape and different positions of a guide arm in the guide track, and
- Figure 7: shows a partial sectional view of a drug delivery device in the area of the needle shield which comprises two snap arms with locking hooks.

Corresponding parts are marked with the same reference symbols in all figures.

### Detailed Description of Preferred Embodiments

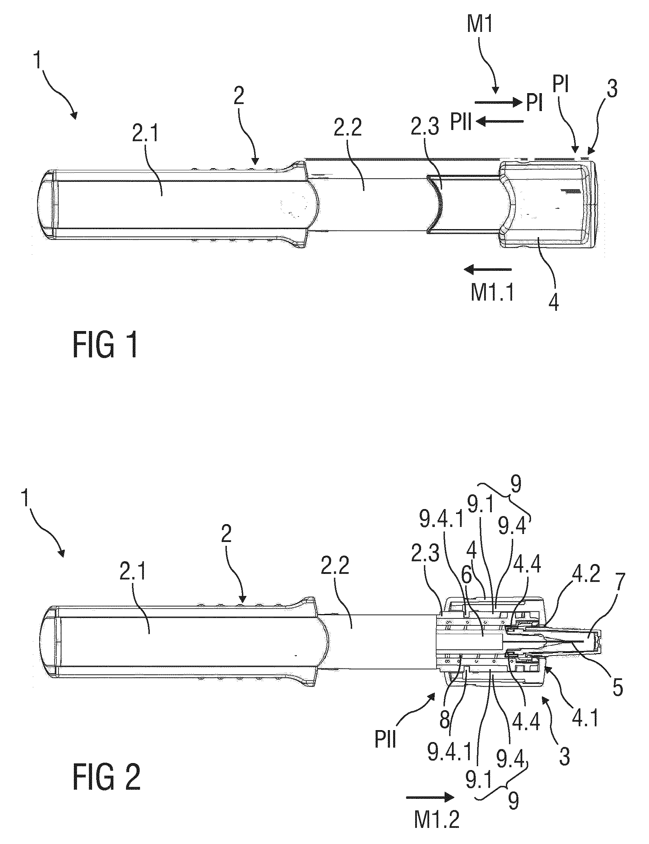

Figure 1 shows a side view of a drug delivery device 1 in a delivery state.

The drug delivery device 1 comprises a housing 2 and a needle safety device 3. The drug delivery device 1 could be an auto-injector with a container, an injection pen or a syringe, e.g. a pre-filled syringe, or another medicament container or any other application with comparable functional principle.

The housing 2 may be designed as a one part or multiple part housing. In the shown embodiment, the housing 2 comprises a back housing part 2.1, a front housing part 2.2 and a support body 2.3.

In the context of this specification, the term "back" or "proximal" end of a component or a device refers to the end closest to the user's hand or furthest away from the delivery or injection site and the term "front" or "distal" or "top" end of a component or a device refers to the end furthest from the user's hand or closest to the delivery or injection site.

The different housing parts may be formed as an injection moulded part, wherein all parts may be formed as an integrated unit or may be separated from each other. In particular, the back housing part 2.1, the front housing part 2.2 and the support body 2.3 are formed as one injection moulded part.

The needle safety device 3 comprises at least a needle shield 4. The needle shield 4 is being mounted on the housing 2 of the drug delivery device 1 in a movable manner according to a first movement M1.1 of a first movement sequence M1. Figure 1 shows the needle shield 4 in an advanced position PI. During the first movement M1.1, the needle shield 4 is movable from the advanced position PI to a retracted position PII (shown in figure 2). In the advanced position PI in the delivery state of the drug delivery device 1, the needle shield 4 is adapted to cover a needle 5 before injection (shown in figure 1).

The needle shield 4 comprises a needle shield end 4.1 adapted to engage an injection site.

As it is shown in figure 2, the housing 2 contains as a container 6 a syringe at which distal end the needle 5 may be fixed. Alternatively, the needle 5 may be removable therefrom, as a matter of design choice.

The needle safety device 3 comprises additionally a protective needle cap 7.

As it can be seen, the needle shield 4 is being movably mounted on the housing 2 by a non-releasable connection, e.g. by a snap-fit connection. A spring 8 biases at one end the needle shield 4 and at the other end the housing 2. The needle shield 4 is adapted to cover the needle 5 before and after injection.

The protective needle cap 7 is being mounted on the needle 5 in a separable manner. The protective needle cap 7 is designed as a protective needle shield so called as a rigid needle shield (shortly named RNS) which covers the needle 5 before use to protect it against damages during transport or travel and to stay it sterile during the assembly process. The protective needle cap 7 is of a typical form and comprises an inner rubber needle shield (not shown) or a soft rubber or rubber like core for easier and safer handling. The inner rubber needle shield is adapted to house and protect the needle 5.

As it is shown in figure 2, in the retracted position PII the needle 5 together with the assembled protective needle cap 7 extends through a distal opening 4.2 of the needle shield 4.

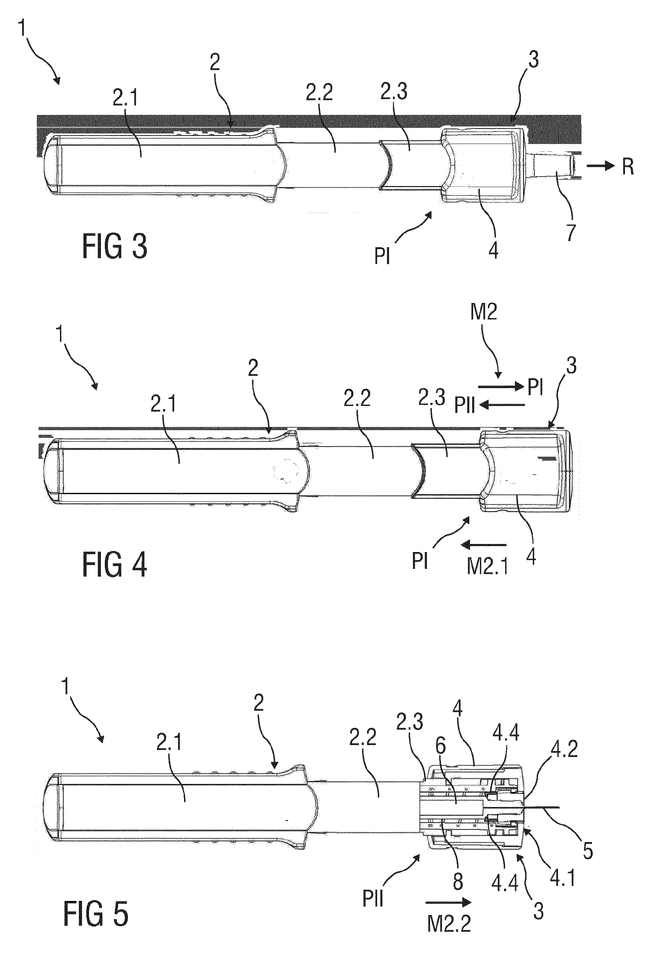

According to the invention, the needle shield 4 is adapted to move in a double movement sequence M1 (shown in the sequence of figures 1 to 3) or M2 (shown in the sequence of figures 3 to 5) between the advanced position PI and the retracted position PII to perform different operations in sequence.

The first movement sequence M1 is shown in the sequence of figures 1 to 3 and represents a first operation of the drug delivery device 1 before an injection, thereby removing the protective needle cap 7.

In the first operation and during the first movement sequence M1 before injection, the needle shield 4 is movable from the advanced position PI (also called starting position) to the retracted position PII (also called intermediate position) and back to the advanced position PI, thereby the needle shield 4 releases the protective needle cap 7.

In particular, during a first movement M1.1 of the first movement sequence M1, the needle shield 4 is adapted to capture the protective needle cap 7 at least in the retracted position PII by a friction connection and/or positive locking connection and/or form-fitting connection.

The needle shield 4 comprises at least one snap arm 4.4 arranged at the needle shield end 4.1 and inwardly directed in such a manner, that the snap arm 4.4 clips over the back end of the protective needle cap 7 during, preferably at the end of the first movement M1.1. Preferably, the needle shield 4 comprises a pair of snap arms 4.4 or a plurality of arms.

Due to the snap-fit connection of the snap arms 4.4 at the back end of the protective needle cap 7, if the needle shield 4 returns back to the advanced position PI during a second movement M1.2 of the first movement sequence M1, the protective needle cap 7 will be taken along and thus away from the needle 5.

As it is shown in figure 3, depending on the length of the needle shield 4 and the length of the protective needle cap 7 as well as of the length of the second movement M1.2, the protective needle cap 7 may be manually removed from the drug delivery device 1 by hand according to an arrow R. Alternatively, the protective needle cap 7 falls off. Thus, the invention allows a removing of the protective needle cap 7 without any additional remover elements.

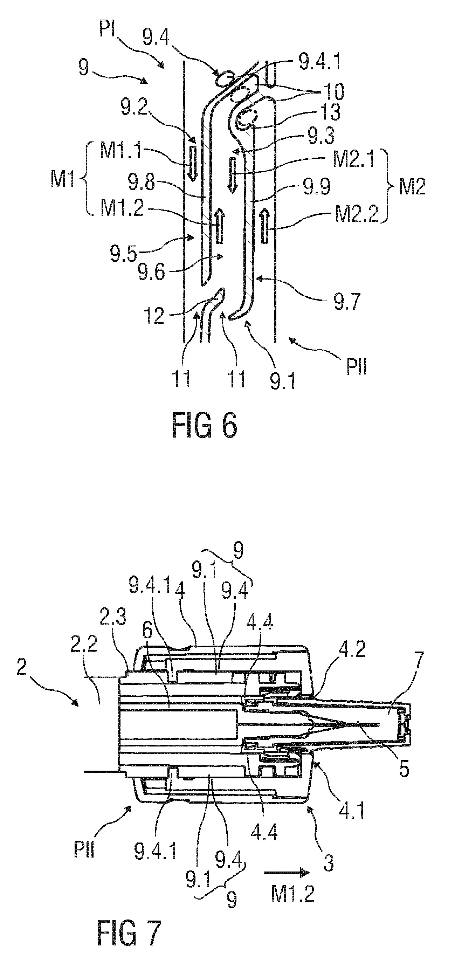

The sequence of figures 4 and 5 shows a second operation with a respective second movement sequence M2 of the needle shield 4. In the second operation and during the second movement sequence M2 and after removing of the protective needle cap 7, the needle shield 4 is movable from the starting or advanced position PI to the retracted position PII (represents a first movement M2.1 of the second movement sequence M2, shown in figures 4 and 5) and back to the advanced position PI (represents a second movement M2.2 of the second movement sequence M2, partially shown in figure 6).

During the first movement M2.1 of the second movement sequence M2, the drug delivery device 1 is placed onto an injection site, thereby the needle shield 4 is movable from the advanced position PI to the retracted position PII so that the needle 5 extends through the distal opening 4.2 of the needle shield 4. During the first movement M2.1, the spring 8 is tensioned by moving of the needle shield 4 into the retracted position PII.

After injection, the drug delivery device 1 will be taken off. Thus, the needle shield 4 is pushed back to the advanced position PI by releasing of spring 8 during the second movement M2.2. After and thus at the end of the second movement M2.2, the needle shield 4 will be securely and permanently fixed in the advanced position PI to permanently cover the needle 5.

Figure 5 shows a possible embodiment of a guiding arrangement 9 for control the double movement sequences M1, M2 of the needle shield 4.

The guiding arrangement 9 is disposed between the housing 2 and the needle shield 4. The guiding arrangement 9 has a guide track 9.1 in labyrinth-shape with different strokes 9.2 to 9.3 for the different movements M1.1 to M2.2 of the movement sequences M1, M2. Preferably, one stroke 9.2 or 9.3 represents one operation and thus one push movement of the needle shield 4 wherein the first stroke 9.2 and the respective first push movement M1.1 of the first sequence M1 represent a release operation for releasing the protective needle cap 7 and the second stroke 9.3 and the respective first push movement M2.1 of the second sequence M2 represent a ready-for-injection operation for allowing injection.

The guiding arrangement 9 comprises further a guide arm 9.4.

As best seen in figures 2 or 5, the drug delivery device 1 comprises two guiding arrangements 9 arranged opposite to each other to the needle safety device 3. The guide track 9.1 is formed in the support body 2.3 of the housing 2 and the guide arm 9.4 is articulated on the needle shield 4 and inwardly directed into the needle shield 4.

The guide arm 9.4 is flexible and comprises an end forming a pin 9.4.1 which cooperates with the guide track 9.1. The guide arm 9.4 is adapted to pivot about a vertical axis.

The guide track 9.1 comprises a plurality of recesses 9.5 to 9.7 which are arranged essentially in parallel to one another and are separated from each other by ribs 9.8 to 9.9 so as to form different stroke guides for the different movements M1.1 to M2.2 of the needle shield 4.

Two of the recesses 9.6 and 9.7 are designed as labyrinth chambers with closed ends 10 and opposite opened ends 11 to form a respective stroke 9.2, 9.3. Due to the one-sided closed recesses 9.5 to 9.7, each recess forms a chamber in which the guide arm 9.4 engages at the closed ends 10 in order to stop the movement of the needle shield 4 with respect to the housing 2 into the advanced position PI.

Accordingly, the opened end 11 of the rib 9.9 and an additional rib 12 arranged in the guide track 9.1 are curved in such a manner that the guide arm 9.4 engages them so that during further movement of the needle shield 4 the guide arm 9.4 flexes by the curved opened end 11 or the rib 12 so that the pin 9.4.1 of the guide arm 9.4 is guided in the area of the opened ends 11 around the end of the ribs 9.8 or 9.9 from one of the recesses 9.5 or 9.6 into an adjacent recess 9.6 or 9.7.

In detail, in the first movement sequence M1 during the first movement M1.1, the guide arm 9.4 is guided from the advanced or start position PI along the recess 9.5 to the opened end 11 and engages the rib 12 in the retracted position PII in which the snap arms 4.4 capture the protective needle cap 7. The guide arm 9.4 is then further guided during the second movement M1.2 around the rib 9.8 into the adjacent recess 9.6 and back to the advanced or intermediate position PI at the closed end 10 of the recess 9.6, thereby the first movement sequence M1 is finished and the protective needle cap 7 is released to fall offs or take off.

During the second movement sequence M2, the guide arm 9.4 is guided from the advanced or intermediate position PI along the recess 9.6 which is slightly curved at the closed end 10 to form a releasable rest position for the pin 9.4.1 of the guide arm 9.4. Due to the curved closed end 10 of the recess 9.6, the guide arm 9.4 is respectively flexed outwards and, by further movement of the needle shield 4, back to engage the rib 9.9 during the first movement M2.1 along the recess 9.6 (=push movement for preparing the injection). The rib 9.9 is slightly curved at the opened end 11 of recess 9.6 so that the guide arm 9.4 flexes again in such a manner that the guide arm 9.4 is guided into the adjacent recess 9.7.

After injection and during the second movement M2.2 the guide arm 9.4 is guided along the recess 9.7. At the closed end 10 of the recess 9.7, the guide arm 9.4 is flexed back into a hook-shaped projection 13 formed in the rib 9.9 so that the pin 9.4.1 of the guide arm 9.4 and thus the needle shield 4 is permanently fixed in the advanced or end position PI after injection and thus after the second movement sequence M2.

The describe labyrinth-shape of the guiding arrangement 9 allows a simple separation of the different operations and push movement sequences M1 and M2 of the needle shield 4.

Furthermore, the needle shield 4 of the drug delivery device 1 sequentially fulfils two operations by two similar succeeding push movement sequences M1, M2, in particular the first push movement sequence M1 for removing the protective needle cap 7 and the second push movement sequence M2 for allowing injection including needle protection before and after injection.

Figure 7 shows the snap arms 4.4 of the needle shield 4 in more detail.

The term "drug" or "medicament", as used herein, means a pharmaceutical formulation containing at least one pharmaceutically active compound,

wherein in one embodiment the pharmaceutically active compound has a molecular weight up to 1500 Da and/or is a peptide, a proteine, a polysaccharide, a vaccine, a DNA, a RNA, an enzyme, an antibody or a fragment thereof, a hormone or an oligonucleotide, or a mixture of the above-mentioned pharmaceutically active compound,

wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,

wherein in a further embodiment the pharmaceutically active compound comprises at least one peptide for the treatment and/or prophylaxis of diabetes mellitus or complications associated with diabetes mellitus such as diabetic retinopathy,

wherein in a further embodiment the pharmaceutically active compound comprises at least one human insulin or a human insulin analogue or derivative, glucagon-like peptide (GLP-1) or an analogue or derivative thereof, or exendin-3 or exendin-4 or an analogue or derivative of exendin-3 or exendin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2,
des Pro36 Exendin-4(1-39),
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(02)25, Asp28] Exendin-4(1-39),
des Pro36 [Trp(02)25, IsoAsp28] Exendin-4(1-39),
des Pro36 [Met(O)14 Trp(02)25, Asp28] Exendin-4(1-39),
des Pro36 [Met(O)14 Trp(02)25, IsoAsp28] Exendin-4(1-39); or

des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(02)25, Asp28] Exendin-4(1-39),
des Pro36 [Trp(02)25, IsoAsp28] Exendin-4(1-39),
des Pro36 [Met(O)14 Trp(02)25, Asp28] Exendin-4(1-39),
des Pro36 [Met(O)14 Trp(02)25, IsoAsp28] Exendin-4(1-39),
wherein the group -Lys6-NH2 may be bound to the C-terminus of the Exendin-4 derivative;
or an Exendin-4 derivative of the sequence
des Pro36 Exendin-4(1-39)-Lys6-NH2 (AVE0010),
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(02)25, Asp28] Exendin-4(1-39)-Lys6-NH2,
H-des Asp28 Pro36, Pro37, Pro38 [Trp(02)25] Exendin-4(1-39)-NH2,
H-(Lys)6-des Pro36, Pro37, Pro38 [Trp(02)25, Asp28] Exendin-4(1-39)-NH2,
H-Asn-(Glu)5-des Pro36, Pro37, Pro38 [Trp(02)25, Asp28] Exendin-4(1-39)-NH2,
des Pro36, Pro37, Pro38 [Trp(02)25, Asp28] Exendin-4(1-39)-(Lys)6-NH2,
H-(Lys)6-des Pro36, Pro37, Pro38 [Trp(02)25, Asp28] Exendin-4(1-39)-(Lys)6-NH2,
H-Asn-(Glu)5-des Pro36, Pro37, Pro38 [Trp(02)25, Asp28] Exendin-4(1-39)-(Lys)6-NH2,
H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2,
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(02)25, Asp28] Exendin-4(1-39)-Lys6-NH2,
H-des Asp28 Pro36, Pro37, Pro38 [Met(O)14, Trp(02)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(02)25, Asp28] Exendin-4(1-39)-NH2,
des Pro36, Pro37, Pro38 [Met(O)14, Trp(02)25, Asp28] Exendin-4(1-39)-(Lys)6-NH2,
H-(Lys)6-des Pro36, Pro37, Pro38 [Met(O)14, Trp(02)25, Asp28] Exendin-4(S1-39)-(Lys)6-NH2,
H-Asn-(Glu)5-des Pro36, Pro37, Pro38 [Met(O)14, Trp(02)25, Asp28] Exendin-4(1-39)-(Lys)6-NH2;
or a pharmaceutically acceptable salt or solvate of any one of the afore-mentioned Exendin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (~150 kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by α, δ, ε, γ, and p. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (C_{H}) and the variable region (V_{H}). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical; only one type of light chain, κ or λ, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

### List of References

- 1: Drug delivery device
- 2: Housing
- 2.1: Back housing part
- 2.2: Front housing part
- 2.3: Support body
- 3: Needle safety device
- 4: Needle shield
- 4.1: Needle shield end
- 4.2: Distal opening
- 4.4: Snap arm
- 5: Needle
- 6: Container
- 7: Protective needle cap
- 8: Spring
- 9: Guiding arrangement
- 9.1: Guide track
- 9.2, 9.3: Stroke
- 9.4: Guide arm
- 9.4.1: Pin
- 9.5-9.7: Recesses
- 9.8, 9.9: Ribs
- 10: Closed ends of recesses
- 11: Opened ends of ribs
- 12: Additional rib
- 13: Hook-shaped projection

- M1: First movement sequence
- M1.1: First movement of the first movement sequence
- M1.2: Second movement of the first movement sequence
- M2: Second movement sequence
- M2.1: First movement of the second movement sequence
- M2.2: Second movement of the second movement sequence

- PI: Advanced position
- PII: Retracted position
- R: Arrow

## Claims

1. Drug delivery device (1), comprising:
- a housing (2) containing and holding a container (6) with a needle (5) attachable at its distal end,
- a needle shield (4) adapted to be movable with respect to the housing (2) between an advanced position (PI) in which the needle (5) is covered and a retracted position (PII) in which the needle (5) extends through an distal opening (4.2) of the needle shield (4), and
- a protective needle cap (7) releasably attached to cover the needle (5) before injection, wherein the needle shield (4) is adapted to move in a double movement sequence (M1, M2) between the advanced position (PI) and the retracted position (PII) to perform different operations in sequence.

2. Drug delivery device (1) according to claim 1, wherein
in a first operation during a first movement sequence (M1) before injection from the advanced position (PI) to the retracted position (PII) and back to the advanced position (PI), the needle shield (4) is adapted to release the protective needle cap (7).

3. Drug delivery device (1) according to claim 1 or 2, wherein
in a second operation after a second movement sequence (M2) from the advanced position (PI) to the retracted position (PII) and back to the advanced position (PI), the needle shield (4) is securely and safely fixed to the housing (2) in the advanced position (PI).

4. Drug delivery device (1) according to one of the preceding claims, wherein
a guiding arrangement (9) is disposed between the housing (2) and the needle shield (4) and guiding the needle shield (4) with respect to the housing (2), wherein the guiding arrangement (9) is adapted to guide the needle shield (4) in the double movement sequence (M1, M2).

5. Drug delivery device (1) according to claim 4, wherein
the guiding arrangement (9) has a labyrinth-shape with different strokes (9.2, 9.3) for different movements.

6. Drug delivery device (1) according to claim 4 or 5, wherein
the guiding arrangement (9) comprises a guide track (9.1) and a guide arm (9.4), wherein the guide track (9.1) is formed in the housing (2) and the guide arm (9.4) is articulated on the needle shield (4).

7. Drug delivery device (1) according to one of the claims 4 to 6, wherein
the guide arm (9.4) is flexible and comprises an end forming a pin (9.4.1) which cooperates with the guide track (9.1).

8. Drug delivery device (1) according to one of the claims 4 to 7, wherein
the guide track (9.1) comprises a plurality of recesses (9.5 to 9.7) which are arranged essentially in parallel to one another and are separated from each other by ribs (9.8, 9.9).

9. Drug delivery device (1) according to claim 8, wherein
each of at least two of the recesses (9.6, 9.7) is designed as a labyrinth chamber with a closed end (10) and an opposite opened end (11) to form a respective stroke (9.2, 9.3).

10. Drug delivery device (1) according to claim 8 or 9, wherein
one of the ends (10) of the recesses (9.5 to 9.7) are closed and the opposite ends (11) are opened in such a manner that the end of the guide arm (9.4) engages one of the closed ends (10) of the recesses (9.5 to 9.7) in the different operations and, during one of the double movement sequences (M1, M2), it is guided from one of the recesses (9.5 to 9.6) through the opened ends (11) into an adjacent recess (9.6 to 9.7).

11. Drug delivery device (1) according to one of the preceding claims 8 to 10,
wherein
the recesses (9.5 to 9.7) are slightly curved in the area of the closed end (10).

12. Drug delivery device (1) according to claim 11, wherein
in the area of a closed end (10) of one of the recesses (9.7), at least one of the respective ribs (9.9) of that recess (9.7) comprises a hooked-shaped projection (13).

13. Drug delivery device (1) according to one of the preceding claims 6 to 12,
wherein
the guide arm (9.4) projects inwards the needle shield (4) and is adapted to pivot about a vertical axis.

14. Drug delivery device (1) according to any of the preceding claims, wherein
a snap arm (4.4) is provided which comprises at its free end a locking hook.

15. Drug delivery device (1) according to one of the preceding claims 4 to 14,
wherein
at least two guide arrangements (9) are provided arranged opposite each other.
